# EUROPEAN PATENT APPLICATION

(11) **EP 1 834 624 A1**
(43) Date of publication of application: **19.09.2007**
(21) Application number: 06702103.0
(22) Date of filing: 05.01.2006
(51) Int. Cl.: A61J 3/00, A61J 3/02, A61K 9/10, A61K 9/14, A61K 31/18, A61K 31/404, A61K 31/42

(54) **MEDICINAL COMPOSITION AND PROCESS FOR PRODUCING THE SAME**

(30) Priority: 07.01.2005 JP 2005003204
(71) Applicant: Eisai R&D Management Co., Ltd., Tokyo 112-8088 (JP)
(72) Inventor: NOMURA, Teruko c/o EISAI CO., LTD., Kakamigahara-shi, Gifu 5016195 (JP); ONAI, Katsumi c/o EISAI CO., LTD., Kakamigahara-shi, Gifu 5016195 (JP); YAMAGUCHI, Takehiro c/o EISAI CO., LTD., Kakamigahara-shi, Gifu 5016195 (JP)
(74) Representative: HOFFMANN EITLE
(86) International application number: PCT/JP2006/300032
(87) International publication number: WO 2006/073154

(57) **Abstract**

A production process which comprises (1) a step in which a raw liquid containing a drug is prepared so that the drug is contained in an amount which exceeds the saturation amount thereof as determined at the temperature to be used in a treatment with a high-pressure homogenizer and is less than the amount which is larger than that drug saturation amount by 1 g per 100 mL of a solvent and that the raw liquid contains substantially no surface modifiers; and (2) a step in which the raw liquid is treated with a high-pressure homogenizer at a pressure of 15-300 MPa while regulating the temperature of the raw liquid so as to exceed the solidifying point of the liquid and be lower than the boiling point thereof. By the process, fine solid particles of a drug and a medicinal composition containing the fine solid drug particles can be obtained.

## Description

### TECHNICAL FIELD

The present invention relates to fine solid particles of a drug substantially containing no surface modifier or a composition containing the fine solid particles, and a production method thereof.

### BACKGROUND ART

Usually, a drug poorly soluble in water has difficulty in blending it with an injection or an oral liquid preparation due to reasons such as low water solubility. Therefore, studies have been made on methods for reducing the size of a poorly-water-soluble drug to a nanometer order for a long time. When the size of a drug is reduced to a nanometer order, the drug increases surface areas while decreasing particle sizes thereof, thereby enabling a markedly increased dissolution rate of the drug. This serves, for example, to improve the absorption or bioavailability of the poorly-soluble drug, or to reduce the amount of solubilizers in liquid formulations which may cause various adverse events. These advantages as above are widely known.

However, it is not easy to produce fine solid drug particles with a nanometer size. In recent years, techniques for yielding fine solid particles have been developed in the field of pulverization typically of pigments and silica. In contrast, reaggregation, decomposition, crystallization, and glass transition of drugs may occur, thereby making it difficult to obtain fine solid particles. Specifically, in the production of fine solid particles, the temperature rises with an increasing energy of pulverization, and this may often invite, for example, decomposition, melting, dissolution in a dispersive medium, and crystal growth due to recrystallization of a drug. Further, fine solid particles once prepared are known to undergo particle growth typically due to reaggregation, crystallization or the like.

The main stream for a conventional production method of fine solid drug particles is a method in which a surface modifier such as a surfactant or a macromolecular substance is used in wet-pulverization using water or a solvent. For example, regarding fine solid particles of less than 400 nm of a drug that is poorly soluble in water, a slurry containing the drug and a surface modifier is treated by a ball mill (Patent Document 1) or by a high-pressure homogenizer (Patent Document 2), so that the surface modifier is adsorbed on the surface of fine solid drug particles and dispersed in a liquid to obtain a suspension. Such a suspension is disclosed as an injection. Further, disclosed is a composition, etc. obtained by adding sucrose to such a suspension and freeze-drying the mixture (Patent Document 3).
Patent Document 1: U.S. Patent No. 5,145,684
Patent Document 2: U.S. Patent No. 5,510,118
Patent Document 3: U.S. Patent No. 5,302,401

### DISCLOSURE OF THE INVENTION

As described above, a surface modifier has been used as an essential component to prevent reaggregation of fine solid particles during or right after the production in the conventional production method. However, a macromolecular substance or a surfactant used as a surface modifier exhibits interaction with other additive in a pharmaceutical composition and thus they may be an impediment to process the pharmaceutical composition. Further, in using an injection containing fine solid drug particles, use of a surface modifier is often restricted due to its safety as an additive. Furthermore, in using an oral liquid agent containing fine solid drug particles, even a trace amount of a surface modifier is disadvantageous in largely affecting the taste or the feeling at dose. Accordingly, there is a demand for developing a production method of fine solid drug particles applicable in various dosage forms while the particles remain unchanged in particle size over time without substantial addition of these surface modifiers and a pharmaceutical composition containing such fine solid drug particles.

A first aspect of the present invention is a method for producing a suspension containing fine solid particles of a drug. The method comprises the steps of: (1) preparing a process liquid so that the process liquid contains the drug in an amount which exceeds a saturation level of the drug at a temperature of treatment by a high-pressure homogenizer and which is less than an amount calculated by adding 1 g of the drug per 100 mL of a solvent to the saturation level, and so that the process liquid contains substantially no surface modifier; and (2) treating the process liquid by the high-pressure homogenizer at a pressure of 15 to 300 MPa while controlling a temperature of the process liquid so as to exceed a solidifying point of the process liquid and be lower than a boiling point thereof.

The first aspect of the present invention has a surprising feature of producing a suspension that contains fine solid particles of a drug and maintains an excellent prolonged dispersibility with no addition of a dispersant. The present inventors have found that an amount of drug particles present in a process liquid is controlled to keep a lower concentration while the process liquid is treated by a high-pressure homogenizer, and thereby the treatment efficiency of the high-pressure homogenizer can be enormously enhanced, thus accomplishing the present invention. This is different from a conventional idea that attempts to treat a process liquid by keeping the drug concentration as high as possible. According to the present invention, the particle size of a drug can be reduced without use of a surface modifier, which has conventionally been an essential component for size reduction by a high-pressure homogenizer.

Further, a second aspect of the present invention is a method for producing fine solid particles of a drug. The method comprises the steps of: (1) preparing a process liquid so that the process liquid contains the drug in an amount which exceeds a saturation level of the drug at a temperature of treatment by a high-pressure homogenizer and which is less than an amount calculated by adding 1 g of the drug per 100 mL of a solvent to the saturation level, and so that the process liquid contains substantially no surface modifier; treating the process liquid by the high-pressure homogenizer at a pressure of 15 to 300 MPa while controlling a temperature of the process liquid so as to exceed a solidifying point of the process liquid and be lower than a boiling point thereof; and (3) removing the solvent from the suspension.

The second aspect of the present invention has a surprising feature of obtaining the fine solid particles of only the drug. According to a conventional method, fine solid particles of a drug are used in conditions wherein the fine solid particles have a surface modifier adsorbed to a surface thereof and is dispersed in a solvent. However, according to the production method of the present invention, fine solid drug particles that substantially contain no component other than the drug can be obtained. Specifically, after a suspension containing fine solid drug particles by the same production method as in the first aspect of the present invention, the solvent is further removed, thereby accomplishing the second aspect. The solvent removal can be performed by a method selected from the group consisting of a suction filtration method, a pressure filtration method, an ultrafiltration method, a spray dry method, a fluidized-bed dry method, a shelved dry method, a freeze dry method, a dry method under reduced pressure, and a vacuum dry method.

Further, a third aspect of the present invention is a method for producing a pharmaceutical composition containing fine solid drug particles of a drug. The method comprises the steps of: (1) preparing a process liquid so that the process liquid contains the drug in an amount which exceeds a saturation level of the drug at a temperature of treatment by a high-pressure homogenizer and which is less than an amount calculated by adding 1 g of the drug per 100 mL of a solvent to the saturation level, and so that the process liquid contains substantially no surface modifier; (2) treating the process liquid by the high-pressure homogenizer at a pressure of 15 to 300 MPa while controlling a temperature of the process liquid so as to exceed a solidifying point of the process liquid and be lower than a boiling point thereof; and (3) mixing a pharmacologically acceptable additive with the suspension.

According to the third aspect of the present invention, a pharmaceutical composition containing fine solid drug particles can be produced using the suspension obtained by the same production method as in the first aspect or fine solid particles obtained by the same production method as in the second aspect, and a pharmacologically acceptable additive. In the third aspect of the present invention, when a solvent contained in a process liquid is not required as a component of the pharmaceutical composition, the method can be provided, which comprises the step of removing a part or all of the solvent from the suspension after the step of obtaining the suspension containing fine solid drug particles. At this time, the step of removing a part or all of the solvent can be performed before or after the step of mixing a pharmacologically acceptable additive with the suspension as long as it is performed after the suspension of the present invention containing fine solid drug particles is produced. Further, the step of removing a part or all of the solvent from the suspension can be performed by a method selected from the group consisting of a suction filtration method, a pressure filtration method, an ultrafiltration method, a spray dry method, a fluidized-bed dry method, a shelved dry method, a freeze dry method, a dry method under reduced pressure, and a vacuum dry method. The third aspect of the present invention enables a pharmaceutical composition to be produced without containing a surface modifier or a solvent, which are unnecessary for the pharmaceutical composition itself. Further, an additive that is incompatible with a surface modifier can be blended in the pharmaceutical composition. Thus, the degree of freedom for formulation design can be significantly enhanced.

In the first, second, and third aspects of the present invention, a process liquid has to contain at least a solvent and a drug. However, if necessary, an additive can be blended that does not function as a surface modifier in the production methods of the present invention. In this case, a mixture containing fine solid drug particles and fine particles of the additive can be obtained. Further, the present invention can provide the production method, which further comprises at least one of the steps of: dry-pulverizing the drug; and treating a pre-process liquid containing the drug and the solvent by an ultrasonic homogenizer or a high-pressure homogenizer prior to the step of preparing the process liquid of the present invention. At this time, the treatment is performed while the drug content of the pre-process liquid is equal to or larger than that of the process liquid of the present invention. That is, using the pre-process liquid containing a dry-pulverized drug and a solvent, the production method can be provided, which comprises the step of treating the pro-process liquid by an ultrasonic homogenizer or a high-pressure homogenizer while the drug content thereof is equal to or larger than that of the process liquid of the present invention.

Further, in the first, second, and third aspects of the present invention, the production method can be provided, wherein the step of preparing the process liquid is to prepare the process liquid so that the process liquid contains the drug in an amount which exceeds a saturation level of the drug at a temperature of treatment by a high-pressure homogenizer and which is less than an amount calculated by adding 0.5 g of the drug per 100 mL of a solvent to the saturation level, and so that the process liquid contains substantially no surface modifier. Further, the production method can be provided, wherein the step to prepare the process liquid so that the process liquid contains the drug in an amount which exceeds a saturation level of the drug at a temperature of treatment by a high-pressure homogenizer and which is less than an amount calculated by adding 0.3 g of the drug per 100 mL of a solvent to the saturation level, and so that the process liquid contains substantially no surface modifier.

A fourth aspect of the present invention is a suspension containing fine solid particles of a drug obtained by a production method comprising the steps of: (a) preparing a process liquid so that the process liquid contains the drug in an amount which exceeds a saturation level of the drug at a temperature of treatment by a high-pressure homogenizer and which is less than an amount calculated by adding 1 g of the drug per 100 mL of a solvent to the saturation level, and so that the process liquid contains substantially no surface modifier; and (b) treating the process liquid by the high-pressure homogenizer at a pressure of 15 to 300 MPa while controlling a temperature of the process liquid so as to exceed a solidifying point of the process liquid and be lower than a boiling point thereof to obtain the suspension containing the fine solid particles of the drug.

Further, a fifth aspect of the present invention is fine solid particles of a drug obtained by a production method comprising the steps of: (a) preparing a process liquid so that the process liquid contains the drug in an amount which exceeds a saturation level of the drug at a temperature of treatment by a high-pressure homogenizer and which is less than an amount calculated by adding 1 g of the drug per 100 mL of a solvent to the saturation level, and so that the process liquid contains substantially no surface modifier; (b) treating the process liquid by the high-pressure homogenizer at a pressure of 15 to 300 MPa while controlling a temperature of the process liquid so as to exceed a solidifying point of the process liquid and be lower than a boiling point thereof to obtain a suspension containing the fine solid particles of the drug; and (c) removing the solvent from the suspension.

Furthermore, a sixth aspect of the present invention is a pharmaceutical composition containing fine solid particles of a drug obtained by a production method comprising the steps of: (a) preparing a process liquid so that the process liquid contains the drug in an amount which exceeds a saturation level of the drug at a temperature of treatment by a high-pressure homogenizer and which is less than an amount calculated by adding 1 g of the drug per 100 mL of a solvent to the saturation level, and so that the process liquid contains substantially no surface modifier; (b) treating the process liquid by the high-pressure homogenizer at a pressure of 15 to 300 MPa while controlling a temperature of the process liquid so as to exceed a solidifying point of the process liquid and be lower than a boiling point thereof to obtain a suspension containing the fine solid particles of the drug; and (c) at least one of the steps of: removing a part or all of the solvent from the suspension; and mixing a pharmacologically acceptable additive with the suspension.

Moreover, the fine solid particles of a drug obtained by the production methods of the present invention preferably have an average particle size of 50 nm to 1000 nm. Almost no changes in the average particle size thereof are observed over time even in a suspension, a pharmaceutical composition, or as independent fine solid particles. This is thus a particularly notable feature.

The present invention can produce a suspension containing fine solid drug particles with a nanometer size without using a surface modifier such as a surfactant or a macromolecular substance. Further, the present invention can provide a production method that enables the processing of a drug without using a surface modifier, that is a novel technique for reducing a particle size of a drug for separately obtaining fine solid drug particles. The fine solid drug particles obtained by the production methods of the present invention are a particulate drug with a particle size of 1 µm or less, and thus it is useful as a pharmaceutical material and widely applicable in developing novel pharmaceutical compositions. For example, the present invention can provide a highly safe injection and an oral liquid preparation excellent in feeling at administration. Furthermore, the present invention can provide a solid composition containing fine solid drug particles or a composition that is easily re-dispersed in water and thus prepared before use. Alternatively, those compositions can be used as a pre-mix material containing fine solid drug particles that can be handled easily and safely while maintaining a particle size of a drug. Moreover, the fine solid drug particles or a pharmaceutical composition containing the same according to the present invention has an excellent storage stability, and thus they are very advantageous as a pharmaceutical raw material or a pharmaceutical in terms of transportation, distribution, or the like.

### BRIEF DESCRIPTION OF THE DRAWINGS

Fig. 1 is a schematic view of a high-pressure homogenizer wherein a reservoir 2, a booster pump 3, and a high-pressure section 1 are connected to each other via a thin tube 5; and
Fig. 2 is a diagram showing changes of average particle sizes of fine solid particles of Drug A in suspensions depending on the pressure at the treatment by a high-pressure homogenizer.

### BEST MODE FOR CARRYING OUT THE INVENTION

Embodiments hereafter are described for exemplifying the present invention, and not intended to limit the present invention. The present invention can be carried out in various forms as long as those forms do not depart from the gist of the present invention.

### (Suspension containing fine solid particles)

A suspension of the present invention containing fine solid particles does not substantially contain a surface modifier such as a surfactant or a macromolecular substance. Herein, the surface modifier is a substance that disperses fine solid particles in a solvent or is added for inhibiting a secondary aggregation. Further, the surface modifier is a substance that can be physicochemically adsorbed on the surface of fine solid particles in a suspension but does not bind chemically to a drug forming fine solid particles. For example, a surfactant, a macromolecular substance, lecithin, and the like have been used as surface modifiers in the conventional techniques. In the present invention, fine solid drug particles can be prepared without substantially containing this surface modifier, and further dispersed stably in a solvent. However, a suspension containing fine solid drug particles may contain, if necessary, an additive that does not function as a surface modifier.

### (Fine solid particles)

Fine solid particles of the present invention are nanometer size particles having an average particle size of 1 µm or less, and they are called as nanoparticles. The fine solid particles of the present invention are preferably in a solid state or in a semisolid state at 1°C to 40°C in a process liquid or a pharmaceutical composition of the present invention, or when the fine solid particles are separated by removing a solvent, and more preferably in a solid state at 1°C to 40°C. The average particle size can be measured using a light scattering method. For example, a dynamic light scattering photometer DLS-7000 or a laser zeta potential analyzer ELS-8000 manufactured by Otsuka Electronics Co., Ltd. may be used as an apparatus that can measure a nanometer size particle size. For measurement, a suspension or a pharmaceutical composition containing fine solid drug particles is mixed with a solvent for measurement and optionally further diluted. The fine solid drug particles of the present invention has an average particle size measured by a light scattering method of 50 nm to 1000 nm, preferably 50 nm to 800 nm, more preferably 100 nm to 400 nm.

### (Drug)

In the present invention, a drug includes a therapeutic agent or a diagnostic agent. The therapeutic agent may be a pharmaceutical containing a synthetic compound, or a biological material such as proteins and peptides. The diagnostic agent may be a radiographic contrast agent or a drug used for other diagnosis. The drug of the present invention is, for example, dispersible in at least one solvent and poorly soluble in that solvent. Herein, the poor solubility means, as solubility of a drug in a solvent, the following terms described in the Japanese Pharmacopoeia 14th edition (hereafter "JP") or the U.S. Pharmacopoeia 24th edition (hereafter, "USP"): slightly soluble (100 mL or more to less than 1000 mL of solvent is required to solve 1 g of a drug at 20 ± 5°C); very slightly soluble (1000 mL or more to less than 10000 mL of solvent is required to solve 1 g of a drug at 20 ± 5°C); and practically insoluble (10000 mL or more of solvent is required to solve 1 g of a drug at 20 ± 5°C). In the production methods of the present invention, a drug has to be present in a solid state in a process liquid in a high-pressure homogenizer. Thus, a drug is preferably a poorly-soluble drug, more preferably a very slightly soluble drug or a practically insoluble drug. A practically insoluble drug is particularly preferable.

### (Kind of drug)

The drug for use in the present invention is not specifically limited in its type and includes antitumor drugs, antibiotics, anti-inflammatory drugs, analgesics, drugs for treating osteoporosis, hypolipidemic drugs, antibacterial drugs, sedative drugs, tranquilizers, antiepileptic drugs, antidepressants, drugs for treating digestive system diseases, drugs for treating allergic diseases, antihypertensive drugs, antiarteriosclerotic drugs, antidiabetic drugs, hormone drugs, and lipid-soluble vitamin preparations. Examples of the antitumor drugs include N-(3-chloro-7-indolyl)-1,4-benzenedisulfonamide, danazol, piposulfam, camptothecin, triiodobenzoate, taxol, doxorubicin hydrochloride, methotrexate, etoposide, 5-fluorouracil, mitoxantrone, mesna, dimesna, aminoglutethimide, tamoxifen, acroline, cisplatinum, carboplatin, and cyclophosphamide. Examples of the antibiotics include amikacin and gentamicin. Examples of the anti-inflammatory drugs are aspirin, phenacetin, acetaminophenone, phenylbutazone, ketophenylbutazone, mefenamic acid, bucolome, benzydamine, mepirizole, tiaramide, tinoridine, prednisolone, hydrocortisone, methylprednisolone, dexamethasone, betamethasone, indomethacin, diclofenac, loxoprofen, ibuprofen, piroxicam, ampiroxicam, and tenoxicam. Examples of the analgesics include Xylocaine, pentazocine, and aspirin. Examples of the drugs for treating osteoporosis include menatetrenone, prostaglandin A1, vitamin D derivatives, sexual hormone derivatives, phenolsulfophthalein, benzothiopyran, and thienoindazole. Examples of the hypolipidemic drugs include clinofibrate, clofibrate, cholestyramine, soysterol, tocopherol nicotinate, nicomol, niceritrol, probucol, and elastase. Examples of the tranquilizers include benzodiazepines such as diazepam, lorazepam, and oxazolam. Examples of the antiepileptic drugs include phenytoin, phenobarbital, carbamazepine, and primidone. Examples of the antidepressants include imipramine, noxiptiline, and phenelzine. Examples of the drugs for treating digestive system diseases include metoclopramide, famotidine, omeprazole, sulpiride, and trepibutone. Examples of the drugs for treating allergic diseases include clemastine fumarate, cyproheptadine hydrochloride, diphenhydramine hydrochloride, methdilazine, clemizole, and methoxyphenamine. Examples of the antihypertensive drugs include nicardipine hydrochloride, delapril hydrochloride, captopril, prazosin hydrochloride, and reserpine. Examples of the antiarteriosclerotic drugs include drugs for inhibiting cholesterol ester transfer protein. The antidiabetic drugs include glymidine, glipzide, glibenclamide, buformin, and metformin. Examples of the hormone drugs include triamcinolone, triamcinolone acetonide, fluocinolone acetonide, hexestrol, methimazole, and estriol. Example of the lipid-soluble vitamin preparations include vitamin A, vitamin D, vitamin E, vitamin K, folic acid, and derivatives thereof. Preferable is a drug poorly soluble in water, and examples thereof include N-(3-chloro-7-indolyl)-1,4-benzenedisulfonamide known as an antitumor drug for inhibiting the growth of cancer cells. N-(3-chloro-7-indolyl)-1,4-benzenedisulfonamide by itself is practically insoluble in water under substantially physiological conditions, i.e., at pH 5 to 7.

### (Solvent and process liquid)

A solvent in the present invention is a medium for processing a drug, and may be a single solvent, or a mixed solvent prepared by blending or mixing two or more kinds of solvents. The solvent is not particularly limited, and it may be a poor solvent hardly solving a drug or a good solvent well solving a drug. Preferably, it is a poor solvent. When a drug is poorly soluble in water, it is more preferably an aqueous medium. Examples thereof include: acid water prepared from acetic acid, various acids, and water; basic water prepared from various bases; or glycol such as glycerin or a mixture liquid of glycol and water. When a drug is not poorly soluble in water, a poor solvent is an organic solvent. Examples thereof include: lower alcohols such as methyl alcohol, ethyl alcohol, and isopropyl alcohol; ketone solvents such as acetone and methyl ethyl ketone; acetonitrile, methyl ether, ethyl ether, or chloroform; and mixtures thereof. When a drug is poorly soluble in water, an organic solvent may be used as a good solvent. In the case of drugs other than that, an aqueous medium is usable. Further, a process liquid in the present invention is a liquid to be treated by a high-pressure homogenizer used to obtain fine solid drug particles, and it contains a solvent and a drug of the present invention. It may optionally contain an additive that does not function as a surface modifier.

### (Pre-treatment)

In the production method of a suspension containing fine solid drug particles in the present invention, pre-treatment may be performed on a drug in order to enhance treatment efficiency for drug size reduction. For example, drug powder is roughly pulverized by a dry mill such as a jet mill or a grinder, and thereafter the obtained product may be used in the production methods of the first, second, and third aspects. Further, a solvent is added to the powder drug, in other word a concentrated liquid with a drug concentration equal to or higher than the process liquid used for high-pressure homogenizer treatment of the present invention or a slurry drug may be pre-treated by a mill or a grinder. Alternatively, a pre-process liquid having a drug content equal to or larger than the drug content of a process liquid of the present invention may be pre-treated by a propeller stirrer, a Homodispa (product name, Mizuho Industrial Co., Ltd.), a Homomixer (product name, Mizuho Industrial Co., Ltd.), a Homojetter (product name, Tokushu Kika Kogyo Kabushiki Kaisha), a Colloid Mill (product name, Tokushu Kika Kogyo Kabushiki Kaisha), an Ultrasonic Homogenizer (product name, Nihon SiberHegner K.K.), a pressure emulsification machine, a high-pressure homogenizer, or the like. These pre-treatment methods may be performed either alone or in combination. The pre-treatment is preferably performed by a dry mill, an ultrasonic homogenizer, or a high-pressure homogenizer. More preferably, a pre-process liquid containing a dry-milled drug and a solvent is prepared so that it has a drug content equal to or larger than the drug content of the process liquid of the present invention, and then the prepared pre-process liquid is treated by an ultrasonic homogenizer or a high-pressure homogenizer.

### (Step for preparing process liquid)

A step of preparing a process liquid in the production methods of the present invention will be exemplified. A drug and a solvent, or a mixture containing the pre-treated drug and a solvent are mixed with each other, and the process liquid is prepared so that the process liquid contains the drug in an amount which exceeds a saturation level of the drug at a temperature of treatment by a high-pressure homogenizer and which is less than an amount calculated by adding 1 g of the drug per 100 mL of the solvent to the saturation level. The process liquid obtained here is poured directly in a reservoir of the high-pressure homogenizer and used for the treatment in the high-pressure homogenizer. The mixing temperature for preparing the process liquid is not particularly limited, but it is desired to adjust the mixing temperature to the temperature at the treatment in the high-pressure homogenizer.

Next, another embodiment will be exemplified. A solvent is poured in a reservoir of a high-pressure homogenizer and circulated in an apparatus while its temperature is controlled. Then, a predetermined amount of a drug, as it is, or suspended in a solvent or dissolved by heating, is poured in the reservoir of the high-pressure homogenizer. The poured drug is circulated in the apparatus thereby to be mixed with the solvent. If necessary, the solvent is further added or a part of the solvent is removed to control the drug content, yielding a process liquid. During the above process, a pressure equal to or lower than that at the treatment in the high-pressure homogenizer of the present invention may be applied to the high-pressure homogenizer. Further, the process liquid is mixed uniformly, and then continuously subjected to high-pressure homogenizer treatment of the present invention. A method for preparing a process liquid of the present invention is not limited to those described above.

### (Drug content)

A drug content in a process liquid of the present invention is such an amount that enables a part of a drug to be present in a solid state in the process liquid at a treatment temperature in a high-pressure homogenizer. Namely, in the present invention, the lower limit of drug content in the process liquid is an amount exceeding a saturation level of the drug at a treatment temperature in the high-pressure homogenizer. The saturation level of a drug means a maximum mass of the drug that can be completely dissolved in the process liquid at that treatment temperature. On the other hand, the upper limit of drug content in the process liquid is not particularly limited as long as it dose not cause a significant reduction in the efficiency for the high-pressure homogenizer treatment. For example, it may be a drug content calculated by adding 1 g of a drug per 100 mL of a solvent to a saturation level in a process liquid at a treatment temperature. Therefore, the drug content in the process liquid of the present invention is more than a saturation level of the drug at the treatment temperature in the high-pressure homogenizer and less than an amount calculated by adding 1 g of the drug per 100 mL of the solvent to the saturation level. The drug content in the process liquid is preferably more than a saturation level of the drug at the treatment temperature in the high-pressure homogenizer and less than an amount calculated by adding 0.5 g of the drug per 100 mL of the solvent to the saturation level.
More preferably, the drug content in the process liquid is more than a saturation level of the drug at the treatment temperature in the high-pressure homogenizer and less than an amount calculated by adding 0.3 g of the drug per 100 mL of the solvent to the saturation level. Still more preferably, the drug content in the process liquid is more than a saturation level of the drug at the treatment temperature in the high-pressure homogenizer and less than an amount calculated by adding 0.1 g of the drug per 100 mL of the solvent to the saturation level.

### (Step of treating by high-pressure homogenizer)

The step of treating a process liquid of the present invention by a high-pressure homogenizer is a step of miniaturizing by a high-pressure homogenizer drug particles in the process liquid having a designated drug content that has been controlled in the previous step. For this treatment, the temperature is, for example, more than a solidifying point of the process liquid and less than a boiling point thereof, and the pressure is 15 to 300 MPa.

### (High-pressure homogenizer)

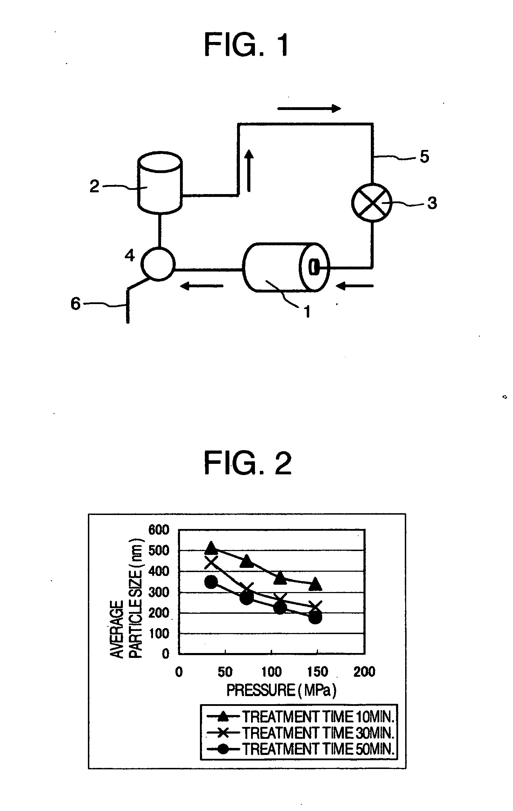

A high-pressure homogenizer used in the present invention is shown in Fig. 1. The high-pressure homogenizer of Fig. 1 comprises a high pressure treatment section 1, a reservoir 2, and a booster pump 3, which are connected via a thin tube 5. A solvent, a drug or a process liquid may be input in the reservoir 2, and thereafter the solvent or the process liquid can be circulated through the thin tube 5. There are provided a flow path switch section 4 and a drain 6. The high-pressure homogenizer may be equipped with an online injector by incorporating the injector at any position of a flow path for the process liquid in the thin tube extending from the reservoir 2 to the high-pressure treatment section 1. The injector is used for controlling a drug content in the process liquid. The injector may be incorporated at any position in the flow path in the thin tube connecting the reservoir 2 and the booster pump 3, or incorporated at any position in the flow path in the thin tube connecting the booster pump 3 and the high-pressure treatment section 1. Further, the injector may be incorporated at any position in the flow path in the thin tube 5 via a joint and/or a mixer. The present invention provides a high-pressure homogenizer equipped with an online injector which can control a drug content in a process liquid by addition of a solvent through the injector immediately before treatment in the high-pressure homogenizer.

The high-pressure homogenizer of the present invention may further comprise a liquid temperature regulator for regulating the temperature of the solvent or the process liquid, which is incorporated in a part or all of the high-pressure treatment section, the reservoir, the booster pump, the online injector, and the thin tube for connecting them to each other. The liquid temperature regulator is not particularly limited, as long as it can regulate the temperature of the solvent or the process liquid at the time of treatment. It is desired that the regulator can serve to remove the heat generated from the high-pressure treatment section, etc. A device for circulating refrigerant around the high-pressure treatment section, for example, may be incorporated.

The high-pressure homogenizer of the present invention has such internal structure of the high-pressure treatment section for example, as to pass the process liquid through a minute orifice to increase the flow rate of the process liquid, and further to allow the high speed flow to collide with a solid wall or allow two high speed flows to collide with each other, thereby obtaining energy for size reduction. In the high-pressure homogenizer of the present invention, the structure of the high-pressure treatment section is not particularly limited. Usable are, for example, a Nanomizer (product name, Yoshida Kikai Co., Ltd.), a Microfluidizer (product name, MFI Corporation, USA), a Piston-gap Homogenizer (product name, EmulsiFlex-C160, Avestin Inc., Canada), an APV-type homogenizer (Invensys Systems Japan, Inc.), a Clear SS5 (product name, M Technique Co., Ltd.), a Niro Soavi homogenizer (Doyei Shoji K.K.), and an Ultimizer (product name, Sugino Machine Limited). A Microfluidizer or a Nanomizer is preferable.

### (Temperature at treatment by high-pressure homogenizer)

The temperature of a process liquid at the treatment by a high-pressure homogenizer of the present invention is not particularly limited. However, usually, the lower limit is a temperature at which the process liquid is not solidified so that a flow path of the high-pressure homogenizer is free from clogging, and the upper limit is a temperature less than a boiling point so that the process liquid does not come to a boil. In other words, the temperature of the process liquid at the treatment exceeds a solidifying point and less than a boiling point. Specifically, when a solvent is an aqueous medium including water as a main solvent, the temperature should be more than 0°C and less than 100°C. The temperature should be preferably more than 0°C and less than 80°C, more preferably 0°C and less than 60°C.

### (Pressure at treatment by high-pressure homogenizer)

When a high-pressure homogenizer is used as a wet-process dispersion machine of the present invention, the treatment pressure is dependent on the ability of machine and thus not particularly limited. However, the treatment pressure is usually 15 to 300 MPa, preferably 30 to 300 MPa, more preferably 70 to 300 MPa. In the treatment by a high-pressure homogenizer, the number of passages of a process liquid through a high-pressure treatment section is not particularly limited, and thus continuous treatment can be performed.

### (Production method of fine solid drug particles)

The second embodiment of the present invention is a production method to separate fine solid drug particles from a suspension containing fine solid drug particles obtained by the production method of the present invention. The fine solid drug particles are obtained by removing all of the solvent from the suspension of the present invention containing fine solid drug particles. The expression "to remove all of the solvent" used herein does not mean to completely remove even a solvent contained in a drug itself, and the expression means "to remove a solvent to a level at which the drug is usable as a drub substance" in the production of a pharmaceutical composition, for example. The step of removing a solvent can be performed either by alone or in combination thereof treatment such as filtration or drying.

### (Filtration method)

A filtration method for removing a solvent from a suspension of the present invention is not particularly limited. Examples of the filtration method include, but are not limited to, a filter filtration method and an ultrafiltration method. Further, these methods may be combined with a drying method to efficiently remove a solvent. Filter filtration methods include a natural filtration method using commercially available filter paper, a suction filtration method using a membrane filter, etc., and a pressure filtration method. In an ultrafiltration method, a drug solution may be also concentrated, and a commercially available ultrafiltration kit (Amicon Co.), for example, may be used. For filtration, a centrifuge machine may be used. A suction filtration method, a pressure filtration method, and an ultrafiltration method are preferable, and an ultrafiltration method is more preferable.

### (Drying method)

For a drying method as a step for removing a solvent from a suspension of the present invention, well-known drying methods can be used either alone or in combination. Exemplary drying methods are, but not limited to, a spray dry method, a fluidized-bed dry method, a shelved dry method, a freeze dry method, a dry method under reduced pressure, and vacuum dry method. Further, they can be used in combination with various filtration methods. In a spray dry method, a suspension of the present invention containing fine solid particles is sprayed by a spray dryer, etc. and dried, yielding fine solid drug particles. Further, in a fluidized-bed dry method, a suspension of the present invention containing fine solid drug particles is sprayed for granulation on powders of lactose, starch or the like, yielding fine solid drug particles. In a freeze dry method, a suspension containing fine solid particles is usually frozen at -20°C to -60°C so that a solvent is removed, followed by drying, thereby enabling the obtainment of fine solid drug particles. In a shelved dry method, a suspension containing fine solid particles is filtered or adsorbed on a filter-shaped support, and then air-dried at about room temperature, yielding fine solid drug particles. In a dry method under reduced pressure or a vacuum dry method, a suspension is filtered and exposed under reduced pressure at an ordinary temperature or under vacuum at an ordinary temperature so that a solvent can be removed. A freeze dry method, a dry method under reduced pressure, a vacuum dry method, and a shelved dry method are preferred. A freeze-dry method is more preferred.

### (Pharmaceutical composition)

A suspension of the present invention containing fine solid particles of a drug may be used directly as a pharmaceutical composition of the present invention. Alternatively, a part or all of a solvent is removed from the suspension, and the resultant product can be used as a pharmaceutical composition of the present invention. Further, the suspension containing fine solid particles of a drug or the fine solid particles of a drug is blended with a pharmacologically acceptable additive; and the obtained mixture can be used as a pharmaceutical composition of the present invention. The pharmaceutical composition of the present invention may be administered to humans and animals orally, rectally, parenterally (intravenously, intramuscularly, or subcutaneously), intracapsularly, intravaginally, intraperitoneally, locally, or buccally or nasally. The pharmaceutical composition of the present invention may be orally administered in the form of tables, powder, fine grain agents, granules, capsules, pills, dry syrups, pastilles, liquids, suspending agents, emulsions, elixirs, syrups, pastilles, or the like. Moreover, it may be parenterally administered in the form of inhalants, suppositories, injections, ointments, patches, cataplasms, ophthalmic ointments, eye drops, nose drops, ear drops, lotions, or the like. The pharmaceutical composition of the present invention may contain fine solid particles of two or more kinds of drugs each having a different concentration.

### (Production method of pharmaceutical composition)

The third aspect of the present invention is a method for producing a pharmaceutical composition, which comprises mixing a pharmacologically acceptable additive with the suspension of the present invention containing fine solid particles of a drug. Further, this method may comprise the step of removing a part or all of a solvent in the suspension after the suspension containing the fine solid particles of the drug is obtained. This step of removing a part or all of the solvent may be performed once or two or more times. As shown in the following flow diagram 1, for example, all of the solvent is removed right after the preparation of the suspension, and fine solid drug particles are separately obtained. Thereafter, a pharmacologically acceptable additive is added to the obtained particles, and a pharmaceutical composition can be obtained using a known method for a pharmaceutical solid formulation. 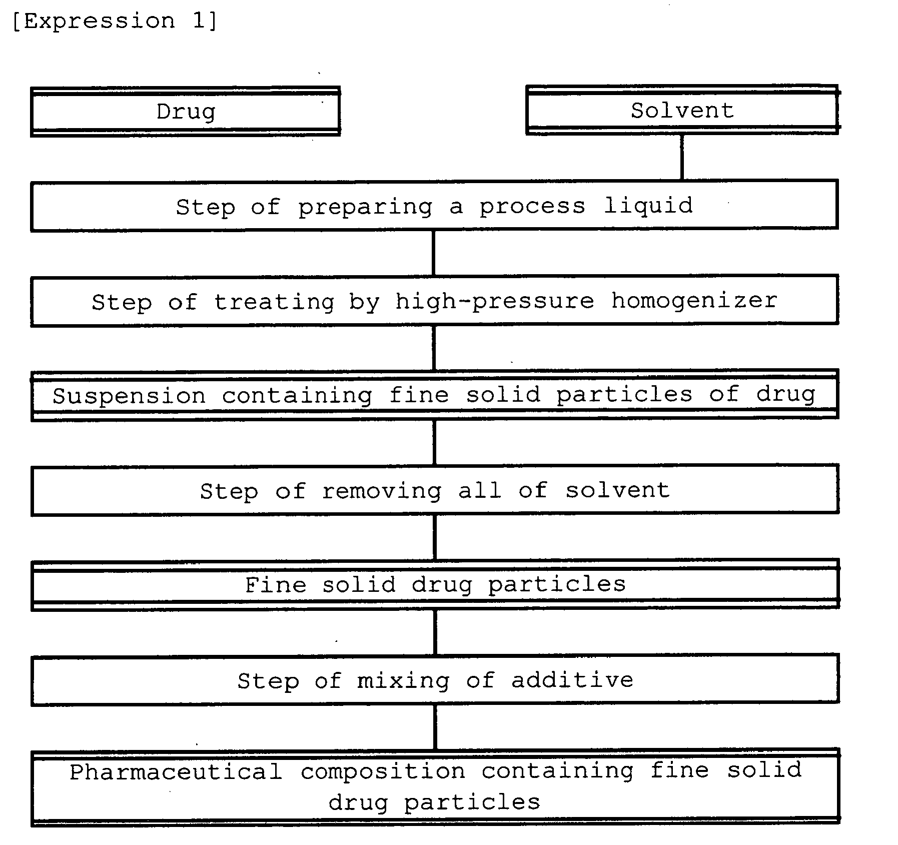 Alternatively, in the case of performing the step of removing the solvent plural times, the method comprises, as shown in the following flow diagram 2, removing a part of the solvent from the suspension containing fine solid particles for concentration, mixing a pharmacologically acceptable additive with the suspension, and then removing the remaining solvent by drying, resulting in the obtainment of a solid pharmaceutical composition or a lyophilized preparation. 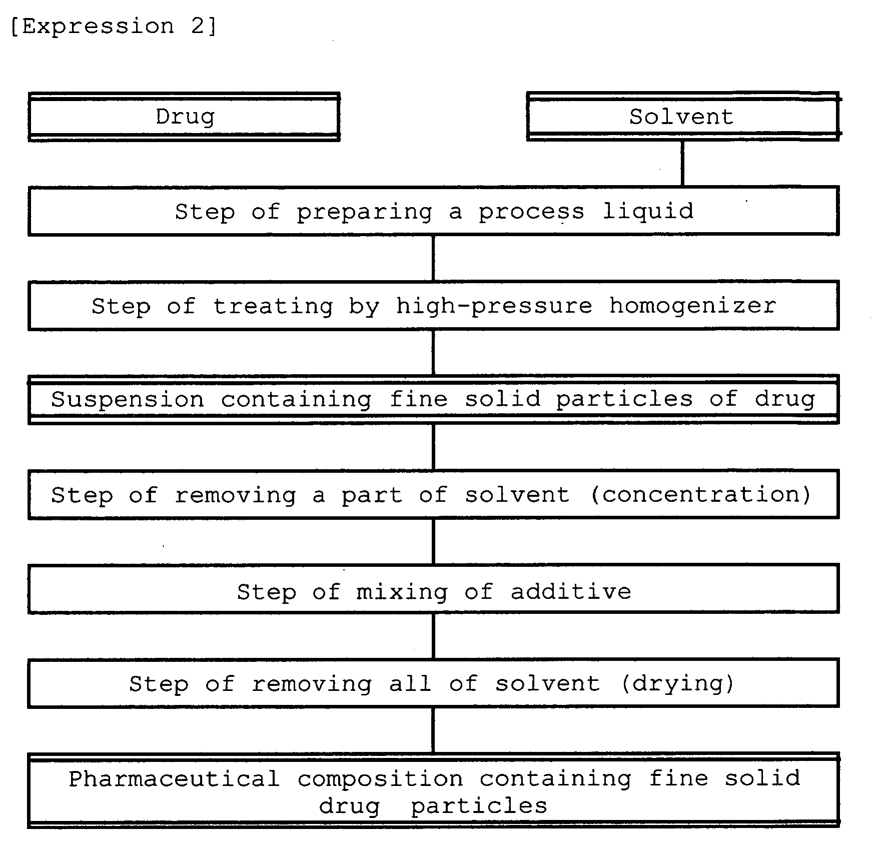 The step of removing a part or all of the solvent can be performed by using various methods of filtration or drying either alone or in combination, which are also used in the step of removing the solvent in the second embodiment of the present invention. Preferably used are a suction filtration method, a pressure filtration method, an ultrafiltration method, a spray dry method, a fluidized-bed dry method, a shelved dry method, a freeze dry method, a dry method under reduced pressure, and a vacuum dry method. More preferably, an ultrafiltration method, a spray dry method, a fluidized-bed dry method, and a freeze dry method are used.

The pharmaceutical composition of the present invention can be formulated as a solid formulation by an ordinary method using a pharmacologically acceptable additive. That is, the solid formulation can be prepared by combination of mixing, granulation, compression, tableting, capsulation and the like. For example, an excipient is added to and mixed with a suspension of the present invention containing fine solid particles or a suspension, from which a part of solvent has been removed, and further the solvent is removed by a spray dry method, thereby enabling the obtainment of a solid composition containing fine drug particles. The solid composition can be used as a pharmaceutical composition such as a powder formulation. Alternatively, addition of a necessary additive and an operation such as granulation or tableting can be performed for producing capsules or tablets containing the fine solid drug particles.

An injection or an oral administration agent of the present invention is formulated directly from the suspension of the present invention by an ordinary method. Alternatively, they are formulated by an ordinary method after a part of solvent is removed for concentration or the solvent is further added for dilution, or after optional addition of a solvent, a solubilizing agent, an isotonic agent, a stabilizer, a soothing agent, a buffer, a suspending agent or the like. In order to formulate a sterile injection, for example, a sterilized drug or the like is used and the production thereof is conducted in a sterile condition, or the production thereof is conducted by a production method including a step of heat sterilization. Further, an injection can be administered intravenously, intradermally, subcutaneously, and intramuscularly. The injection of the present invention is preferably an injection that is prepared before use. For example, an isotonic agent such as sucrose, glucose, D-mannitol, and sodium chloride is optionally added to the suspension of the present invention containing fine solid particles and dried by a freeze dry method to obtain a pharmaceutical composition. The obtained pharmaceutical composition may be used as an injection that is prepared before use.

An agent for external use of the present invention is formulated directly from the suspension of the present invention by an ordinary method. Alternatively, it is formulated as an ointment, a cream agent, a suppository, a patch, a lotion or the like by an ordinary method after a part of solvent is removed for concentration or the solvent is further added for dilution, or after optional addition of a solvent, a base material, an emulsifier, a thickener, a preservative, a stabilizer or the like. Alternatively, like an injection, the agent for external use may be a lotion that is prepared before use.

### (Additive)

An additive of the present invention is a pharmacologically acceptable substance and is used in a pharmaceutical composition of the present invention. Specific examples of the additive are as follows. Exemplary excipients include D-mannitol, lactose, saccharose, corn starch, crystalline cellulose, calcium hydrogenphosphate dehydrate, and precipitated calcium carbonates. Exemplary binders include povidone, dextrin, hydroxypropylcellulose, hydroxypropyl methylcellulose, methylcellulose, polyvinyl alcohol, sodium carboxymethylcellulose, and gelatinized starches. However, the excipient and binder are not limited thereto. Exemplary lubricants include hardened oils, stearic acid, magnesium stearate, and sodium stearyl fumarate. Exemplary disintegrators include low substitution degree hydroxypropyl cellulose, carmellose, sodium starch glycolate, and crospovidone. However, the lubricant and disintegrator are not limited thereto. Further, exemplary coating agents include: cellulose derivatives such as hydroxypropyl cellulose, hydroxypropyl methylcellulose, and carmellose sodium; acrylic polymers such as ethyl acrylate-methyl methacrylate copolymer dispersion and methacrylate copolymer L; synthetic polymer substances such as polyvinylpyrrolidone, polyvinyl alcohol, and polyoxyethylene polyoxypropylene glycol; and natural polymer substances such as gelatine and gum Arabic. Exemplary plasticizers include dioctyl adipate, triethyl citrate, triacetin, glycerin, concentrated glycerin, and propylene glycol. Exemplary suspending agents or emulsifiers include lecithin, sucrose fatty acid ester, polyglycerol fatty acid ester, polyoxyethylene hydrogenated castor oil, polysorbate, and polyoxyethylene-polyoxypropylene copolymers. Exemplary flavoring agents include menthol, peppermint, and orange oil. Exemplary sugar-coated agents include saccharose, lactose, starch syrup, precipitated calcium carbonates, gum Arabic, and carnauba wax. Exemplary fluidizers include silicon dioxide hydrate, light anhydrous silicic acid, calcium stearate, talc, and corn starch. Exemplary coloring agents include: tar colors such as Food Yellow No. 4, Food Yellow No. 5, Food Red No. 2, Food Red No. 102, Food Blue No. 1, Food Blue No. 2 (indigocarmine), and Food Yellow No. 4 aluminum lake; Yellow ferric oxide; iron oxide red (colcothar); titanium oxide; talc; and riboflavin. Examples of the solvent include, but not limited to, water, ethyl alcohol, isopropyl alcohol, ethyl acetate, propylene glycol, peanut oil, castor oil, sesame oil, glycerin, and polyethyleneglycol.

A suspension containing fine solid particles of the present invention is prepared, for example, by the following method. A drug (for example, N-(3-chloro-7-indolyl)-1,4-benzenedisulfonamide) poorly-soluble in water is pulverized by a jet mill in advance and 50 mg of the drug is dispersed in 50 mL of water. The obtained dispersion is vigorously shaken, yielding a process liquid. Next, the process liquid is treated by a high-pressure homogenizer. For example, it is treated by a nanomizer at a pressure of 148 MPa for 30 minutes, enabling the obtainment of a suspension containing fine solid particles of the drug. Further, from the suspension containing fine solid particles of the drug, a solvent is removed by concentration, drying, or the like, so that fine solid particles of the drug can be obtained. Furthermore, 10 mL of 10% mannitol aqueous solution is added to 5 mL of the suspension, and the mixture is freeze-dried, enabling the production of a lyophilized preparation.

Hereafter, the present invention will be described in detail by referring to Examples, but the present invention is not limited thereto. Further, in a pharmaceutical composition used were an additive and a reagent that comply with an official compendium such as Japanese Pharmacopoeia, Japanese Pharmaceutical Excipients 2003 (JPE), and Japanese Pharmaceutical Codex 1997 (JPC).

### (Measurement method of average particle size)

An average particle size was measured to evaluate Examples of the present invention or Comparative Example. The measurement was conducted by means of a dynamic light scattering method using DLS-7000 (Otsuka Electronics Co., Ltd.) or ELS-8000 (Otsuka Electronics Co., Ltd.), and a cumulant diameter was adopted as an average particle size. A suspension containing fine solid particles was mixed with purified water and stirred, and the obtained mixture was used as a sample to be measured.

### (Examples 1 to 3 and Comparative Example 1)

25 mg of N-(3-chloro-7-indolyl)-1,4-benzenedisulfonamide (hereafter referred to as Drug A) pulverized by a jet mill was placed in a sample bottle with a twist-off cap, and 50 mL of purified water was added thereto. The resultant mixture was vigorously shaken and mixed, yielding a process liquid for a high-pressure homogenizer treatment. The process liquid was poured in a reservoir of a nanomizer, passed through a flow path, and subjected to size reduction in a high-pressure treatment section. For the size reduction, the temperatures of a jacket for the high-pressure treatment section and the flow path of the process liquid were controlled to be 0°C (the process liquid had a temperature of 17°C) and the treatment was conducted at a pressure of 148 MPa for 50 minutes, and solid particles of Drug A (average particle size of 181 nm) (Example 1) could be obtained. In the same manner as above, 50 mg of Drug A (Example 2) pulverized by a jet mill and 150 mg thereof (Example 3) were treated to yield suspensions containing fine solid particles of Drug A having an average particle size of 177 nm and 554 nm, respectively. Further, 250 mg of Drug A was treated in the same manner as above, yielding a suspension containing particles of Drug A (Comparative Example 1).

### (Test Example 1: analysis of drug content in process liquid)

During the production of Examples 1 to 3 and Comparative Example 1, time-sequential sampling was conducted to measure average particle sizes of Drug A in the Examples and Comparative Example by DLS-7000. At that time, the concentration of Drug A in measured samples was 0.07 mg/mL. Results are shown in Table 1. Here, a drug content in a process liquid is expressed in a mass of Drug A relative to 1 mL of solvent. In the cases of Example 1 (drug content: 0.5 mg) and Example 2 (drug content: 1 mg), 10-minute of treatment time allowed them to have an average particle size of 500 nm or less. Further, the particle sizes decreased as the treatment time increased, and became 200 nm or less after 50-minute of treatment time. Example 2 (drug content: 3 mg) decreased its average particle size over time, and had an average particle size of about 550 nm after 50-minute of treatment time. However, it was found that Comparative Example 1 (drug content: 5 mg) could not decrease its particle size to 1000 nm or less even after 50-minute of treatment time. Here, Drug A had a saturation solubility of 0.0001 g/mL or less in water at a process liquid temperature of 17°C.

**[Table 1]**

| Treatment time (min.) | Drug content relative to 1 mL of solvent | | | |
|---|---|---|---|---|
| | Example 1 | Example 2 | Example 3 | Comparative Example 1 |
| | 0.5 mg | 1 mg | 3 mg | 5 mg |
| 0 | 2773 | 3263 | - - - | - - - |
| 10 | 365 | 339 | 434 | 2252 |
| 20 | 243 | 271 | 603 | 2000 |
| 30 | 209 | 227 | 879 | 3359 |
| 50 | 181 | 177 | 554 | 4227 |

### (Examples 4 to 6)

In the same manner as in Example 2, suspensions containing fine solid particles of Drug A were produced with different treatment temperatures. For the preparation, the jacket temperature for the high-pressure treatment section and the flow path was controlled to be 10°C (Example 4), 20°C (Example 5), and 40°C (Example 6), and accordingly, the temperature of the process liquid at the treatment by the high-pressure homogenizer was 26°C, 35°C, and 52°C, respectively. In all the Examples, suspensions containing fine solid particles of the drug having a nanometer size were produced efficiently. However, when the temperature for the high-pressure treatment section and the flow path was not controlled for the same formulation as Example 2, a process liquid increased its temperature to 96°C and had too large particles to be measured by DLS-7000. This is because when the temperature exceeds a temperature at which the concentration of the process liquid reaches to a saturation solubility, the drug is dissolved. Then, the drug is not treated as solid particles in a suspension. Further, when the suspension is cooled after the treatment, crystal deposition or crystal growth may occur. Thus, it is considered that fine solid particles of interest could not be obtained.

### (Test Example 2)

Time-sequential sampling of Examples 4 to 6 was conducted in the same manner as for Test Example 1 to measure particle sizes by DLS-7000. Results are shown in Table 2. In the temperature range of from 17°C to 52°C, there was less temperature influence and fine solid particles having almost the same average particle size could be obtained.

**[Table 2]**

| Treatment time (min.) | Temperature of process liquid at treatment | | | |
|---|---|---|---|---|
| | Example 2 | Example 4 | Example 5 | Example 6 |
| | 17°C | 26°C | 35°C | 52°C |
| 0 | 3263 | - - - | - - - | 3424 |
| 10 | 339 | 349 | 290 | 320 |
| 30 | 227 | 193 | 209 | 238 |
| 50 | 177 | 189 | 191 | 224 |

### (Examples 7 to 9)

Suspensions containing fine solid particles of Drug A were produced in the same manner as in Example 2. The treatment was conducted at a pressure of 110 MPa (Example 6), 73 MPa (Example 7), and 35 MPa (Example 8).

### (Test Example 3)

Time-sequential sampling of Examples 7 to 9 was conducted in the same manner as for Test Example 1 to measure particle sizes by DLS-7000. Results are shown in Table 3. As a result, the pressure range from 35 MPa to 148 MPa enabled the production of a suspension containing fine solid particles of the drug having a nanometer size. Further, Fig. 2 shows changes of average particle size relative to pressure. As a result, a higher pressure reduces an average particle size. It was suggested that even when the treatment was conducted at a pressure lower than 35 MPa for Example 9, fine solid particles were obtainable.

**[Table 3]**

| Treatment time (min.) | Treatment pressure | | | |
|---|---|---|---|---|
| | Example 2 | Example 7 | Example 8 | Example 9 |
| | 148MPa | 110MPa | 73MPa | 35MPa |
| 0 | 3263 | - - - | - - - | - - - |
| 10 | 339 | 370 | 450 | 513 |
| 30 | 227 | 263 | 316 | 442 |
| 50 | 177 | 225 | 271 | 349 |

### (Example 10)

40 mg of Drug A pulverized by a jet mill was placed in a sample bottle with a twist-off cap, and 40 mL of purified water was added thereto. The resultant mixture was vigorously shaken and mixed, yielding a process liquid. The process liquid was poured in a reservoir of a nanomizer, and treated at a pressure of 148 MPa up to 50 minutes while the high-pressure section and the solvent flow path were controlled to have a temperature of 0°C, producing a suspension containing fine solid particles of the drug.

### (Test Example 4)

To confirm the stability of fine solid particles of Drug A, the suspension obtained in Example 10 was concentrated and further allowed to stand at room temperature for one day. Then, the average particle size of fine solid particles in the suspension was measured by DLS-7000.

### <Concentration method>

The suspension was concentrated using an ultrafiltration kit (Amicon Co.) up to about 1.7 to 3.3 times of the concentration immediately after the production.

### <Concentration measurement of Drug A>

The concentration of Drug A was measured by liquid chromatography method under the following conditions.

### Preparation of sample solution

About 1 mL of suspension was measured by a 10 mL-volumetric flask, and 1 mL of methanol was added to and dissolved in the suspension. Then, the mixture was diluted to a fixed volume with a dilute solution of water/methanol/phosphoric acid (550/450/1).

### Preparation of standard solution

50 mg of Drug A was measured by a 50 mL-volumetric flask and dissolved by 30 mL of methanol. After dissolution, the mixture was diluted to a fixed volume with a dilute solution of water/methanol/phosphoric acid (550/450/1), and the stirred. 5 mL of this stock solution was measured by a 50 mL-volumetric flask and diluted to a fixed volume with the above-mentioned dilute solution, and then stirred.

### HPLC conditions

Wavelength: 223 nm, Column (recommended): ODS-A, 4.6 × 75 mm, 3 µm, YMC, Mobile phase A: 60%, B: 40% (Mobile phase A: water/acetonitrile/phosphoric acid (950/50/1); Mobile phase B: water/acetonitrile/phosphoric acid (100/900/1)), Column temperature: a constant temperature around 40°C, Flow rate: 1.5 mL/min, Injection volume: 5 µL.

Measurement results of Test Example 4 are shown in Table 4. It was confirmed that the average particle size of the suspension of Example 10 remained unchanged after a lapse of one day since the preparation and stable with no dispersant. Further, even when the suspension was concentrated from an initial concentration to three-time concentration, the particle size was unchanged. Furthermore, the particle size remained unchanged after a lapse of one day since the concentration. It was confirmed that the suspension of the present invention was such a stable system that no secondary aggregation was caused by concentration.

**[Table 4]**

| Measurement on preparation day | | Measurement after one day |
|---|---|---|
| Drug concentration (mg/mL) | Average particles size (nm) | Average particles size (nm) |
| 0.49 | 216 | 206 |
| 0.84 | 213 | - - - |
| 1. 64 | 213 | 219 |

### (Example 11 to Example 14: lyophilized preparation)

40 mg of Drug A pulverized by a jet mill was placed in a sample bottle with a twist-off cap, and 40 mL of purified water was added thereto. The resultant mixture was vigorously shaken and mixed, yielding a process liquid. The process liquid was poured in a reservoir of a nanomizer, and pulverized at a pressure of 148 MPa up to 50 minutes while the high-pressure section and the solvent flow path were controlled to have a temperature of 0°C, producing a suspension containing fine solid particles of Drug A. The concentration of this solution was measured by an HPLC, and the concentration was 0.39 mg/mL. 4.5 mL of 10% lactose solution or 4.5 mL of 5% mannitol solution was added to and mixed well with 4.5 mL of the solution. Then, 1 mL of each solution was poured in a vial and lyophilized, yielding lyophilized preparations containing as an isotonic agent lactose (Example 11) and mannitol (Example 12), respectively. Further, suspensions containing fine solid particles of Drug A produced in Examples 11 and 12 (having a concentration of Drug A of 0.39 mg/mL) were poured in an ultrafiltration kit and adjusted so that they had a concentration of 1.12 mg/mL. 4.0 mL of 10% lactose solution or 4.0 mL of 5% mannitol solution was added to and mixed with 4.0 mL of this concentrated suspension. Then, 1 mL of each solution was poured in a vial and lyophilized, yielding lyophilized preparations containing as an isotonic agent lactose (Example 13) and mannitol (Example 14), respectively.

### (Test Example 5)

The lyophilized preparations prepared in Examples 11 to 14 were used to study influence of lyophilization on fine solid particles of Drug A. A sample of the lyophilized preparation was reconstituted with 1 mL of water for injection and an average particle size of fine solid particles of Drug A in the lyophilized preparation was measured by DLS-7000. Results are shown in Table 5. Table 5 also shows average particle sizes of fine solid particles of the drug in the suspensions before lyophilization, and average particle sizes of find solid particles measured after the reconstituted solutions were stored at room temperature for one day. As a result, no remarkable difference in average particle sizes before and after lyophilization was observed regardless of the concentrations of Drug A in the suspensions before lyophilization. Accordingly, compositions containing stable fine solid particles of the drug were obtained. From the experimental results, no influence of the isotonic agent was observed, and reaggregation of fine solid particles and aggregation growth thereof attributable to the lyophilization were not observed.

**[Table 5]**

| | Drug A concentration in suspension | Isotonic agent | Average particle size (nm) | | |
|---|---|---|---|---|---|
| | | | Suspension | Lyophilized preparation (right after reconstitution) | Reconstitution (after one day) |
| Example 11 | 0.39 mg/mL | Lactose | 188 | 200 | 205 |
| Example 12 | 0.39 mg/mL | Mannitol | 187 | 205 | 207 |
| Example 13 | 1.12 mg/mL | Lactose | 183 | 198 | 204 |
| Example 14 | 1.12 mg/mL | Mannitol | 192 | 213 | 227 |

### (Test Example 6: storage stability test)

The lyophilized preparations prepared in Examples 11 to 14 were stored in a thermostat bath at a temperature of 40°C for 2 weeks and 1 month. Thereafter, the average particle sizes of fine solid particles of Drug A were measured by DLS-7000, and compared with the average particle sizes at initial production stages of Examples. Evaluation results are shown in Table 6. After 1 month-storage at 40°C, the particle sizes of the reconstituted suspensions were unchanged. It was confirmed that the pharmaceutical compositions of the present invention stably preserved their fine solid particles of the drug regardless of storage conditions. From the experimental results, it was confirmed that the stability of fine solid particles of the drug was less affected by the concentration of the drug or the kind of additive in the pharmaceutical compositions.

**[Table 6]**

| Storage at 40°C | Average particle size (nm) | | |
|---|---|---|---|
| | Initial stage | 2 weeks | 1 month |
| Example 11 | 200 | 200 | 205 |
| Example 12 | 205 | 205 | 206 |
| Example 13 | 202 | 198 | 210 |
| Example 14 | 213 | 213 | 220 |

### (Examples 15 to 19)

Glibenclamide (Wako Pure Chemical Industries, Ltd.: saturation solubility in water: 5.9 µg/mL (22°C), 9.9 µg/mL (37°C)), which was a poorly-soluble drug in water, was pulverized in a mortar as pretreatment. 25 mg of the pulverized product was placed in a sample bottle with a twist-off cap, and 50 mL of purified water was added thereto. The resultant mixture was vigorously shaken and mixed, yielding a process liquid. The process liquid was poured in a reservoir of a nanomizer, and treated at a pressure of 148 MPa for 50 minutes while the high-pressure section and the solvent flow path were controlled to have a temperature of 0°C, producing a suspension containing fine solid particles of the drug (Example 15). Likewise, 50 mg (Example 16), 100 mg (Example 17), 150 mg (Example 18), and 250 mg (Example 19) of glibenclamide pulverized in a mortar were each suspended in 50 mL of water for injection, and treated by a nanomizer under the same conditions as in Example 15, yielding suspensions containing fine solid particles of glibenclamide.

### (Test Example 7)

During the production of the suspensions of Examples 15 to 18, time-sequential sampling was conducted in the same manner as in Test Example 1 to measure average particle sizes by DLS-7000 (Table 7). In the case of glibenclamide, use of 0.5 g or less of drug content to be treated per 100 mL of solvent enabled the production of fine solid particles of the drug with no addition of a dispersant.

**[Table 7]**

| Treatment time (min.) | Drug content per 1 mL of solvent | | | | |
|---|---|---|---|---|---|
| | Example 15 | Example 16 | Example 17 | Example 18 | Example 19 |
| | 0.5 mg | 1 mg | 2 mg | 3 mg | 5 mg |
| 0 | 1607 | 2520 | 1557 | 3758 | - - - |
| 5 | 593 | 600 | 602 | 771 | 530 |
| 10 | 471 | 452 | 566 | 570 | 719 |
| 20 | 346 | 366 | 444 | 426 | 629 |
| 50 | 421 | 339 | 477 | 917 | 895 |

### (Examples 20 to 23)

12.5 mg of danazol (Sigma; 1 µg/mL of saturation solubility in water) was placed in a sample bottle with a twist-off cap, and 50 mL of purified water was added thereto. The resultant mixture was vigorously shaken and mixed, yielding a process liquid for treatment by a high-pressure homogenizer. The process liquid was poured in a reservoir of a nanomizer, and miniaturized at a high-pressure section through a flow path. For the size reduction, the temperature of a jacket for the high-pressure section and the flow path of the process liquid were controlled to be 0°C (the process liquid had a temperature of 16°C), and the treatment was conducted at a pressure of 148 MPa for 50 minutes, enabling the obtainment of a suspension of fine solid particles of danazol (Example 20). In the same manner as above, 25 mg of danazol (Example 21), 50 mg thereof (Example 22), and 150 mg thereof (Example 23) each pulverized in a mortar were suspended in 50 mL of purified water, and miniaturized by a nanomizer under the same conditions as Example 20.

### (Test Example 8)

During the production of Examples 20 to 23, time-sequential sampling was conducted to measure average particle sizes of danazol particles by ELS-8000. Measurement results immediately after the production were shown in Table 8. Here, a drug content in a process liquid is expressed in a mass of danazol relative to 1 mL of solvent. In the cases of Example 1 (drug content: 0.25 mg) and Example 2 (drug content: 0.5 mg), 5-minute of treatment time enabled the obtainment of fine particles with an average particle size of 1000 nm or less. Further, the average particle sizes decreased as the treatment time increased, and after 50-minute of treatment time, the suspensions of Examples 1 and 2 had an average particle size of 403 nm and 255 nm, respectively. Example 3 (drug content: 1 mg) and Example 4 (drug content: 3 mg) had 1000 nm or less of fine particles after the treatment time of 5 minutes to 30 minutes. However, in the case of Example 4, after 60-minute treatment of the suspension, fine particles with an average particle size of 1000 nm or less were not obtained. From the above results, in the case of danazol, use of 0.3 g or less of drug content to be treated per 100 mL of solvent enabled the production of fine solid particles of the drug with no addition of a dispersant.

**[Table 8]**

| Treatment time (min.) | Drug content per 1 mL of solvent | | | |
|---|---|---|---|---|
| | Example 20 | Example 21 | Example 22 | Example 23 |
| | 0.25 mg | 0.5 mg | 1 mg | 3 mg |
| 0 | ----- | 9365 | ----- | ----- |
| 5 | 924 | 900 | 636 | 917 |
| 10 | 677 | 610 | 533 | 630 |
| 20 | 549 | 481 | 567 | 626 |
| 30 | ----- | ----- | 651 | 704 |
| 50 | 403 | 255 | ----- | 1372 |

Further, the average particle sizes of suspensions sampled at a treatment time of 20 minutes were measured by ELS-8000 after a lapse of one day since the production. As a result, the suspensions of all the Examples exhibited almost no changes of average particle size after the lapse of one day since the production. It was confirmed that the suspensions were stable with no dispersant.

**[Table 9]**

| Treatment time (min.) | Drug content per 1 mL of solvent | | | |
|---|---|---|---|---|
| | Example 20 | Example 21 | Example 22 | Example 23 |
| | 0.25 mg | 0.5 mg | 1 mg | 3 mg |
| Initial stage | 549 | 481 | 567 | 626 |
| After one day | 645 | 398 | 675 | 675 |

## Claims

1. A method for producing a suspension containing fine solid particles of a drug, comprising the steps of:
(1) preparing a process liquid so that the process liquid contains the drug in an amount which exceeds a saturation level of the drug at a temperature of treatment by a high-pressure homogenizer and which is less than an amount calculated by adding 1 g of the drug per 100 mL of a solvent to the saturation level, and so that the process liquid contains substantially no surface modifier; and
(2) treating the process liquid by the high-pressure homogenizer at a pressure of 15 to 300 MPa while controlling a temperature of the process liquid so as to exceed a solidifying point of the process liquid and be lower than a boiling point thereof.

2. A method for producing fine solid particles of a drug, comprising the steps of:
(1) preparing a process liquid so that the process liquid contains the drug in an amount which exceeds a saturation level of the drug at a temperature of treatment by a high-pressure homogenizer and which is less than an amount calculated by adding 1 g of the drug per 100 mL of a solvent to the saturation level, and so that the process liquid contains substantially no surface modifier;
(2) treating the process liquid by the high-pressure homogenizer at a pressure of 15 to 300 MPa while controlling a temperature of the process liquid so as to exceed a solidifying point of the process liquid and be lower than a boiling point thereof; and
(3) removing the solvent from the suspension.

3. The production method according to claim 2, wherein the step (3) is performed by a method selected from the group consisting of a suction filtration method, a pressure filtration method, an ultrafiltration method, a spray dry method, a fluidized-bed dry method, a shelved dry method, a freeze dry method, a dry method under reduced pressure, and a vacuum dry method.

4. A method for producing a pharmaceutical composition containing fine solid particles of a drug, comprising the steps of:
(1) preparing a process liquid so that the process liquid contains the drug in an amount which exceeds a saturation level of the drug at a temperature of treatment by a high-pressure homogenizer and which is less than an amount calculated by adding 1 g of the drug per 100 mL of a solvent to the saturation level, and so that the process liquid contains substantially no surface modifier;
(2) treating the process liquid by the high-pressure homogenizer at a pressure of 15 to 300 MPa while controlling a temperature of the process liquid so as to exceed a solidifying point of the process liquid and be lower than a boiling point thereof; and
(3) mixing a pharmacologically acceptable additive with the suspension.

5. The production method according to claim 4, further comprising the step of removing a part or all of the solvent from the suspension after the step (2).

6. The production method according to claim 5, wherein the step of removing a part or all of the solvent from the suspension is performed by a method selected from the group consisting of a suction filtration method, a pressure filtration method, an ultrafiltration method, a spray dry method, a fluidized-bed dry method, a shelved dry method, a freeze dry method, a dry method under reduced pressure, and a vacuum dry method.

7. The production method according to any one of claims 1 to 6, wherein the step (1) is to prepare a process liquid so that the process liquid contains the drug in an amount which exceeds a saturation level of the drug at a temperature of treatment by a high-pressure homogenizer and which is less than an amount calculated by adding 0.5 g of the drug per 100 mL of a solvent to the saturation level, and so that the process liquid contains substantially no surface modifier.

8. The production method according to any one of claims 1 to 6, wherein the step (1) is to prepare a process liquid so that the process liquid contains the drug in an amount which exceeds a saturation level of the drug at a temperature of treatment by a high-pressure homogenizer and which is less than an amount calculated by adding 0.3 g of the drug per 100 mL of a solvent to the saturation level, and so that the process liquid contains substantially no surface modifier.

9. The production method according to any one of claims 1 to 8, wherein the fine solid particles of the drug has an average particle size of 50 nm to 1000 nm.

10. The production method according to any one of claims 1 to 9, further comprising at least one of the steps of: dry-pulverizing the drug; and treating a pre-process liquid containing the drug and the solvent by an ultrasonic homogenizer or a high-pressure homogenizer, prior to the step (1).

11. A suspension containing fine solid particles of a drug obtained by a production method comprising the steps of:
(a) preparing a process liquid so that the process liquid contains the drug in an amount which exceeds a saturation level of the drug at a temperature of treatment by a high-pressure homogenizer and which is less than an amount calculated by adding 1 g of the drug per 100 mL of a solvent to the saturation level, and so that the process liquid contains substantially no surface modifier; and
(b) treating the process liquid by the high-pressure homogenizer at a pressure of 15 to 300 MPa while controlling a temperature of the process liquid so as to exceed a solidifying point of the process liquid and be lower than a boiling point thereof to obtain the suspension containing the fine solid particles of the drug.

12. Fine solid particles of a drug obtained by a production method comprising the steps of:
(a) preparing a process liquid so that the process liquid contains the drug in an amount which exceeds a saturation level of the drug at a temperature of treatment by a high-pressure homogenizer and which is less than an amount calculated by adding 1 g of the drug per 100 mL of a solvent to the saturation level, and so that the process liquid contains substantially no surface modifier;
(b) treating the process liquid by the high-pressure homogenizer at a pressure of 15 to 300 MPa while controlling a temperature of the process liquid so as to exceed a solidifying point of the process liquid and be lower than a boiling point thereof to obtain a suspension containing the fine solid particles of the drug; and
(c) removing the solvent from the suspension.

13. A pharmaceutical composition containing fine solid particles of a drug obtained by a production method comprising the steps of:
(a) preparing a process liquid so that the process liquid contains the drug in an amount which exceeds a saturation level of the drug at a temperature of treatment by a high-pressure homogenizer and which is less than an amount calculated by adding 1 g of the drug per 100 mL of a solvent to the saturation level, and so that the process liquid contains substantially no surface modifier;
(b) treating the process liquid by the high-pressure homogenizer at a pressure of 15 to 300 MPa while controlling a temperature of the process liquid so as to exceed a solidifying point of the process liquid and be lower than a boiling point thereof to obtain a suspension containing the fine solid particles of the drug; and
(c) at least one of the steps of: removing a part or all of the solvent from the suspension; and mixing a pharmacologically acceptable additive with the suspension.
